# EUROPEAN PATENT APPLICATION

(11) **EP 1 318 145 A1**
(43) Date of publication of application: **11.06.2003**
(21) Application number: 01955672.9
(22) Date of filing: 10.08.2001
(51) Int. Cl.: C07D 231/16, C07D 231/14

(54) **PROCESSES FOR THE PREPARATION OF PYRAZOLE COMPOUNDS**

(30) Priority: 16.08.2000 JP 2000246688; 27.09.2000 JP 2000293395
(71) Applicant: NIPPON SODA CO., LTD., Chiyoda-ku, Tokyo 100-8165 (JP)
(72) Inventor: YAGIHARA,T. Odawara Res.Cen.Nippon Soda Co., Ltd., Odawara-shi, Kanagawa250-0280 (JP); IMAGAWA, Tsutomu Takaoka Plant, Nippon Soda Co.Ltd, Takaoka-shi, Toyama 938-8507 (JP); SUGA,S. Odawara Research Center,Nippon Soda Co.Ltd, Odawara-shi, Kanagawa 250-0280 (JP); HATANO,M. Odawara Research Cen.NipponSoda Co.Ltd., Odawara-shi, Kanagawa 250-0280 (JP); SHIBATA,Y. Odawara Research Cen.Nippon Soda Co.Ltd, Odawara-shi, Kanagawa 250-0280 (JP); OOKUCHI,T. Odawara Research Cen.Nippon Soda Co.Ltd, Odawara-shi, Kanagawa 250-0280 (JP); KASAHARA, Isamu Takaoka Plant, Nippon Soda Co.Ltd, Takaoka-shi, Toyama 938-8507 (JP)
(74) Representative: Wibbelmann, Jobst, Dr., Dipl.-Chem.
(86) International application number: JP0106925
(87) International publication number: WO02014284

(57) **Abstract**

The invention relates to a process (1) for the preparation of 4-cyanoacetylpyrazoles of the general formula [I] 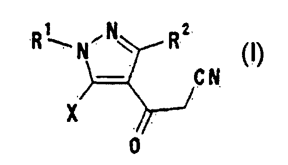 (wherein R1 is C1-6 alkyl; R2 is C1-6 alkyl or C1-6 halo-alkyl; and X is hydrogen or C1-6 alkyl) which comprises using a pyrazolecarboxylic acid halide and a cyanoacetate ester, a process (2) for the preparation of the same which comprises using a pyrazolecarboxylic acid and a cyanoacetate ester, a process (3) for the preparation which comprises using a pyrazolecarbonitrile, and a process (4) for the preparation which comprises using a haloacetylpyrazole. Further, the invention also provides an industrially advantageous process for preparing pyrazolecarboxylic acids useful as intermediates in the above processes.

## Description

### Technological Fields:

The present invention relates to processes for the preparation of novel 4-cyanoacetylpyrazoles useful as intermediates for producing agricultural chemicals, drugs and the like, and to processes for the preparation of pyrazolecarboxylic acids useful as intermediates in the above processes.

### Background Art:

4-Cyanoacetylpyrazoles are useful as intermediates for producing agricultural chemicals and drugs, such as insecticidal active components described in Japanese Patent Laid-open No. Hei 11-269173 and others.

Japanese Patent Laid-open No. Hei 11-269173 describes pyrazole compounds represented by the following formula 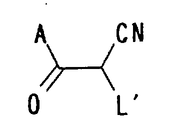 as actual examples of compounds represented by the formula 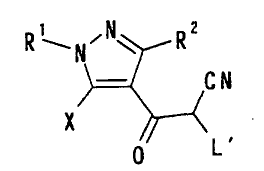 (wherein, L' is a leaving group such as halogen). It has however no concrete descriptions of processes for the preparation of the compounds. Besides, there are no disclosures of analogous compounds and their production processes.

Japanese Patent Laid-open No. Hei 6-87821 has disclosed cyanoacetylpyrazoles as intermediates for producing acrylonitrile-type fungicides, with no descriptions of actual production processes.

The following are examples of known processes for the preparation of pyrazol-4-carboxylic acids from pyrazol-4-carboxyaldehydes.
(1) J.L. Huppatz: Aust. J. Chem. 36, 135-147 (1983) describes a method for producing a carboxylic acid by reacting 5-chloro- 1 ,3-dimethylpyrazolyl-4-carboaldehyde with potassium permanganate, as shown in the following reaction equation. This process is not, however, an excellent method in terms of industrial production because of treatment of manganese dioxide produced in the reaction, troublesome reaction operations and others. 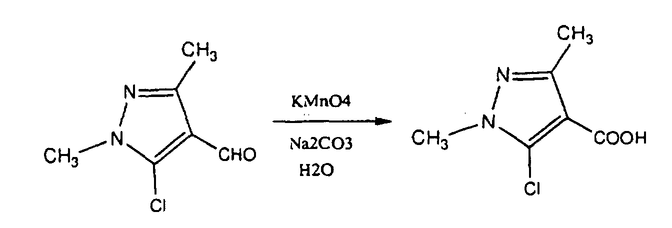
(2) In Japanese Patent Laid-open No. 2-42061, a carboxylic acid is produced using a salt of oxo acid, such as sodium chlorite, as an oxidizing agent in the presence of a heavy metal salt such as nickel or cobalt salt. A problem is that the heavy metal salt is not used at a catalytic amount.
(3) Japanese Patent Laid-open No. Hei 4-120059 describes a process for the preparation of a carboxylic acid by reacting a substituted pyrazol-4-carboaldehyde with sodium chlorite in a two-phase system of water and an organic solvent under an acidic condition in the presence of hydrogen peroxide and a phase transfer catalyst.
   It is presumed that, in the above process, sodium chlorite works as an oxidizing agent Treatments after the reaction are complicated and post-treatments of the reaction mixture also become time-consuming due to the presence of additives.
(4) Japanese Patent Laid-open No. Hei 7-70076 describes an improved method of Process (3): a substituted pyrazol-4-carboaldehyde is reacted with sodium chlorite and hydrogen peroxide in an aqueous solvent under an acidic condition in the presence or absence of a phase transfer catalyst.

### Disclosure of the Invention:

It is an object of the present invention to provide industrially advantageous processes for the preparation of 4-cyanoacetylpyrazoles useful as intermediates for producing agricultural chemicals and drugs, from easily available compounds.

The inventors studied in earnest to solve the above problems, and found a process (1) for the preparation of 4-cyanoacetylpyrazoles of Formula [I] 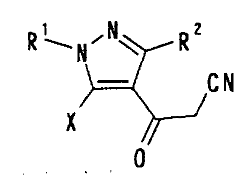 (wherein, R¹ is alkyl having 1 to 6 carbons, R² is alkyl having 1 to 6 carbons or haloalkyl having 1 to 6 carbons, and X is hydrogen or alkyl having 1 to 6 carbons) which comprises using a pyrazolecarboxylic acid halide and a cyanoacetate ester, a process (2) for the preparation of the same which comprises using a pyrazolecarboxylic acid and a cyanoacetate ester, a process (3) for the preparation of the same which comprises using a pyrazolecarbonitrile; and a process (4) for the preparation of the same which comprises using a haloacetylpyrazole.

Furthermore, an industrially advantageous process for preparing pyrazolecarboxylic acids useful as intermediates in the above processes has also been found.

Firstly, the present invention provides a process for the preparation of a compound of Formula [I] which comprises a process for producing a compound represented by Formula[IV] 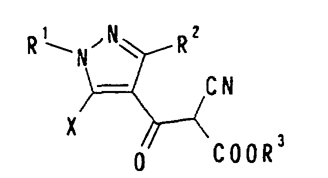 (wherein, R¹, R² and X are as defined above and R³ is alkyl having 1 to 6 carbons) by reacting a pyrazolecarboxylic acid halide represented by Formula [II] 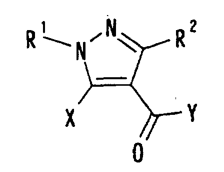 (wherein, R¹, R² and X are as defined above, and Y is halogen) with a cyanoacetate ester represented by Formula [III] 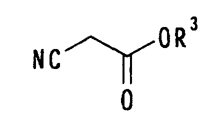 (wherein, R³ is as defined above) in the presence of a base, and a process for the hydrolysis and decarboxylation of the obtained compound of Formula [IV].

Secondly, the present invention provides a process for the preparation of a compound of Formula [I] which comprises a process for producing a compound of [IV] by reacting a pyrazolecarboxylic acid of Formula [V] 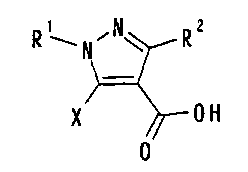 (wherein, R¹, R² and X are as defined above) with a cyanoacetate ester of Formula [III] in the presence of a condensing agent, and a process for the hydrolysis and decarboxylation of the obtained compound of Formula [TV].

Thirdly, the present invention provides a process for the preparation of a compound of Formula [I] which comprises a process for producing a compound of [VII] 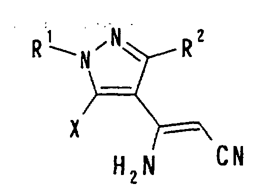 (wherein, R¹, R² and X are as defined above) by reacting a pyrazolecarbonitrile of Formula [VI] 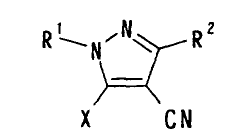 (wherein, R¹, R² and X are as defined above) with acetonitrile in the presence of a base, and a process for the hydrolysis of the obtained compound of Formula [VII].

Fourthly, the present invention provides a process for the preparation of a compound of Formula [I] which comprises reacting a haloacetylpyrazole of Formula [VIII] 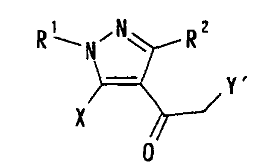 (wherein, R¹, R² and X are as defined above, and Y' is halogen) with a cyanating agent.

Furthermore, the fifth of the present invention is a process for the preparation of a pyrazolecarboxylic acid of Formula [X] 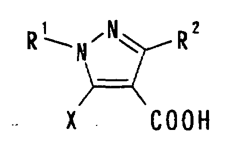 which is an intermediate used in the above processes, by reacting a compound represented by Formula [IX] 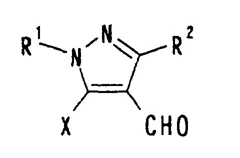 (wherein, R¹, R² and X are as defined above) with hydrogen peroxide in the presence of a base.

### Forms to Implement the Invention:

Processes for the preparation of 4-cyanoacetylpyrazoles of the present invention are described in the following. 4-Cyanoacetylpyrazoles of the present invention are compounds represented by Formula [I]. In Formula [I], R¹ is straight-chain or branched alkyl having 1 to 6 carbons. Its actual examples include methyl, ethyl, n-propyl, isopropyl. n-butyl, sec-butyl, t-butyl, n-pentyl, isopentyl, 2-methylbutyl, 1-methylbutyl, n-hexyl, isohexyl, 3-methylpentyl, 2-methylpentyl, 1-methylpentyl, n-heptyl and isoheptyl. A preferred group is methyl or ethyl.

R² is alkyl having 1 to 6 carbons or haloalkyl having 1 to 6 carbons. Actual examples of alkyl having 1 to 6 carbons include those cited for R¹ in the above. Examples of haloalkyl having 1 to 6 carbons include fluoromethyl, chloromethyl, bromomethyl, 2,2,2-trichloroethyl, bromoethyl, fluoro-n-propyl, difluoromethyl, pentafluoroethyl, chlorodifluoromethyl, trifluoromethyl, dichloromethyl, trichloromethyl, 2,2,2-trifluoroethyl and 2,2,2-trichloroethyl. Of them, methyl, ethyl or trifluoromethyl is preferred.

X is hydrogen, halogen or alkyl having 1 to 6 carbons. Fluorine, chlorine or bromine is exemplified as halogen. Examples of alkyl having 1 to 6 carbons include methyl, ethyl, n-propyl, isopropyl, n-butyl, sec-butyl, t-butyl, n-pentyl, isopentyl, 2-methylbutyl, 1-methylbutyl, n-hexyl, isohexyl, 3-methylpentyl, 2-methylpentyl, 1-methylpentyl, n-heptyl and isoheptyl.

The 4-cyanoacetylpyrazoles may be produced according to one of the following Processes 1 to 4.
A process 1, which comprises converting a pyrazolecarboxylic acid to an corresponding acid halide and reacting a pyrazolecarboxylic acid halide with a cyanoacetic acid ester, followed by hydrolysis and decarboxylation of the resulting product.
A process 2, which comprises reacting a pyrazolecarboxylic acid with a cyanoacetic acid ester in the presence of a condencing agent, followed by decarboxylation of the resulting product.
A process 3, which comprises reacting a pyazolecarbonitrile with acetonitrile in the presence of base, followed by hydrolsis of the resulting product.
A process 4, which comprises reacting a halopyrazole with a cyanating agent

Each process is described in detail in the following. 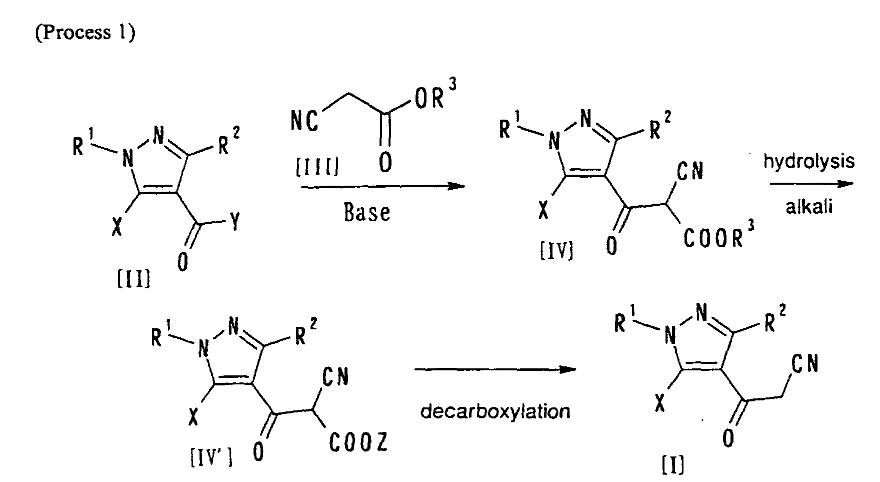 (In the equation, R¹, R² and X are the same as those of Formula [I], Y is halogen such as fluorine, chlorine or bromine, and Z is alkali metal.)

First, a pyrazolecarboxylic acid halide [II] is reacted with a cyanoacetic acid ester [III] in an organic solvent in the presence of a base, preferably a base and a catalyst, to give a compound of Formula [IV].

In the cyanoacetate ester of Formula [III], R³ is alkyl having 1 to 6 carbons. Its actual examples include methyl, ethyl, n-propyl, isopropyl, n-butyl, sec-butyl, t-butyl, n-pentyl, isopentyl, 2-methylbutyl, 1-methylbutyl, n-hexyl, isohexyl, 3-methylpentyl, 2-methylpentyl, 1-methylpentyl, n-heptyl and isoheptyl.

An amount of a cyanoacetate ester [III) is preferably in a range from 1 to 3 moles to a mole of pyrazolecarboxylic acid halide.

Examples of organic solvents used for the reaction include ethers such as diethyl ether, tetrahydrofuran (THF),1,3-dimethoxyethane and 1,4-dioxane; amides such as N,N-dimethylformamide (DMF), N,N-dunethylacetamide and hexamethylphosphate triamide (HMPT); aromatic hydrocarbons such as benzene, toluene, xylene and ethyl benzene; halogenated hydrocarbons such as dichloromethane, chloroform and carbon tetrachloride; and dimethyl sulfoxide (DMSO) and acetonitrile. These can be used alone or in the form of a mixed solution of two or more. Of them, ethers such as diethyl ether, tetrahydrofuran (THF),l,3-dimethoxyethane and 1,4-dioxane; amides such as N,N-dimethylfomamide (DMF), N,N-dimethylacetamide and hexamethylphosphate triamide (HMPT); and aprotic polar solvents such as dimethyl sulfoxide and acetonitrile are preferably used.

An organic or inorganic base can be used as a base. Its actual examples include metal alkoxides such as sodium ethoxide, sodium methoxide, potassium t-butoxide, magnesium methoxide and magnesium ethoxide; organic bases such as triethy(amine, diisopropylethylamine, pyridine, 1,4-diazabicyclo[2.2.2]octane (DABCO), 4-dimethylaminopyridine (DMAP) and 1,4-diazabicyclo[5.4.0]und-7-ene (DBU); organic lithium compounds such as n-butyl lithium, sec-butyl lithium and t-butyl lithium; lithium amides such as lithium diisopropylamide (LDA) and lithium bistrimethylsilylamide; alkali metal hydroxides such as sodium hydroxide and potassium hydroxide; alkali metal carbonates such as sodium carbonate and potassium carbonate; and metal hydrides such as sodium hydride and calcium hydride. These bases can be used alone or combining two or more. An amount of a base used is arbitrarily chosen in a range between 1 and 3 moles to a mole of pyrazolecarboxylic acid halide.

In this reaction, use of a catalyst such as DMAP may make the reaction proceed promptly and efficiently. An amount of a catalyst used is arbitrarily chosen in a range where the reaction proceeds efficiently. Usually it is in a range between 0.001 and 1 mole to a mole of pyrazolecarboxylic acid halide. The reaction proceeds smoothly at -10 Cto 200 C,preferably at room temperature to 100 C.

Then, the obtained product is hydrolyzed in a solvent to give a compound of Formula [IV']. The hydrolysis is usually carried out in an aqueous alkali solution. Examples of alkalis used include potassium hydroxide and sodium hydroxide.

An amount of an alkali used for the hydrolysis is arbitrarily chosen in a range between 0.5 moles and excessive moles, preferably between 1 and 3 moles, to a mole of pyrazolecarboxylic acid halide.

Preferred solvents used for the hydrolysis are water, or mixed solvents of water and an organic solvent compatible with water, such as water-alcohol and water-THF. The hydrolysis reaction can be carried out at a temperature from room temperature to the boiling point of the solvent used.

Finally, an acid is added to the obtained aqueous alkaline solution for decarboxylation to give a 4-cyanoacetylpyrazole of Formula [I].

Actual examples of acids used for the decarboxylation include inorganic acids such as hydrochloric acid and sulfuric acid and organic acids such as acetic acid. Preferred are strongly acidic inorganic acids such as concentrated hydrochloric acid and concentrated sulfuric acid.

An amount of an acid used is arbitrarily chosen in a range between 0.01 moles and excessive moles to a mole of pyrazolecarboxylic acid halide. The decarboxylation reaction proceeds smoothly at room temperature to 100 C. 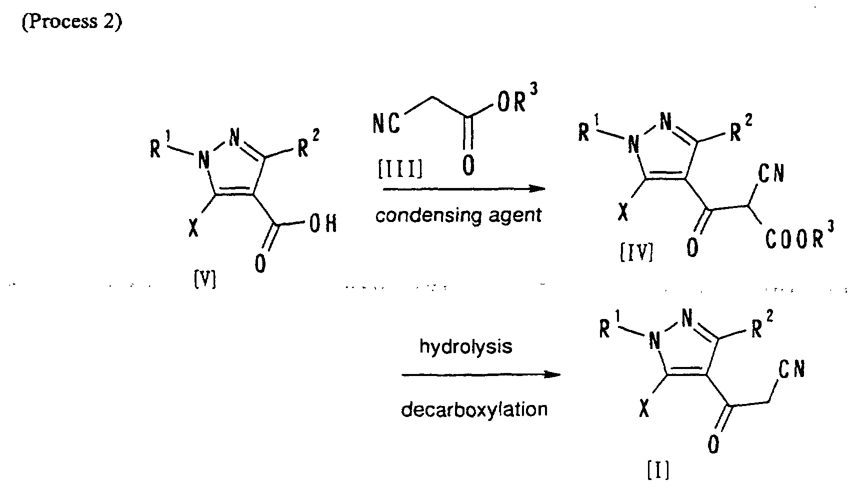 (In the equation, R¹, R², R³ and X are as defined above.)

First, a pyrazolecarboxylic acid [V] is reacted with a cyanoacetic acid ester [III], using a condensing agent, in an organic solvent in the presence of a base.

There are no restrictions on condensing agents used for the reaction, if they can be used as dehydrating agents. Actual examples include diethylcyanophosphonate and dicyclohexylcarbodiimide. An amount of a condensing agent used is arbitrarily chosen in a range between 0.5 moles and excessive moles, preferably between 1 and 3 moles, to a mole of pyrazolecarboxylic acid.

An organic base is preferably used as a base. Its actual examples include triethylamine, diisopropylethylamine, pyridine, DMAP, DBU and DABC. An amount of base used is in a range between 0.5 moles and excessive moles, preferably between 1 and 3 moles, to a mole of pyrazolecarboxylic acid.

Examples of reaction solvents used include ethers such as 1,2-dimethoxyethane, diethyl ether, 1,4-dioxane and THF; amides such as DMF, N,N-dimethylacetamide and HMPT; halogenated hydrocarbons such as dichloromethane, chloroform and carbon tetrachloride; aromatic hydrocarbons such as benzene, toluene, xylene and ethyl benzene; and DMSO and acetonitrile. Of them, ethers such as 1,2-dimethoxyethane, diethyl ether, 1,4-dioxane and THF; amides such as DMF, N,N-dimethylacetamide and HMPT; and aprotic polar solvents such as DMSO and acetonitrile are preferably used. They can be used alone or combining two or more.

The condensation reaction is usually carried out at a temperature from 0 C to the boiling point of the solvent used, preferably room temperature to the boiling point of the solvent used.

Then, the product of the condensation reaction is decarboxylated in an organic solvent in the presence of an acid catalyst, to give the target compound of Formula [I].

Examples of acid catalysts used include p-toluenesulfonic acid, p-toluenesulfonic acid hydrate, benzene sulfonic acid, sulfuric acid, hydrochloric acid and hydrogen bromide. An amount of an acid catalyst used is usually in a range between 0.001 and 1 mole to a mole of pyrazolecarboxylic acid halide.

The decarboxylation reaction is carried out in a solvent including a protic solvent such as water, methanol or ethanol; or an aromatic hydrocarbon such as benzene or toluene. The reaction proceeds smoothly at a temperature from 0 Cto the boiling point of the solvent used. 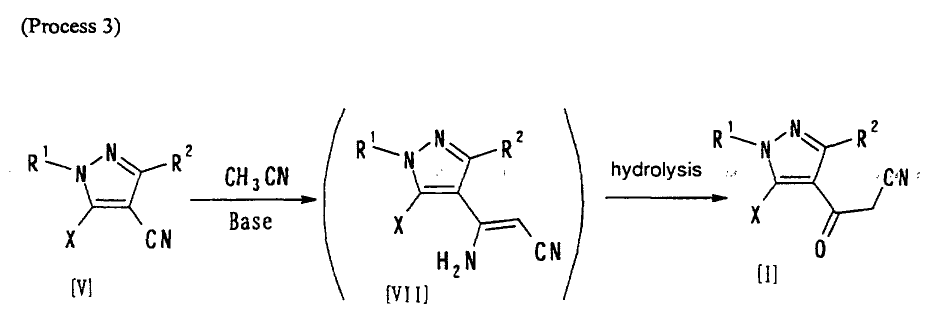 (In the equation, R¹, R² and X are as defined above.)

First, a pyrazolecarbonitrile [VI] is reacted with acetonitrile in the presence of a base to give an adduct [VII].

For this reaction, acetonitrile itself can be used as a solvent. Other usable solvents include ethers such as 1,2-dimethoxyethane (DME), diethyl ether, 1,4-dioxane and THF; aromatic hydrocarbons such as benzene, toluene, xylene and ethyl benzene; amides such as DMF, N,N-dimethylacetamide and HMPT; and DMSO.

An inorganic or organic base can be used as a base. Its actual examples include alkali metal alkoxides such as sodium ethoxide, sodium methoxide and potassium t-butoxide; organic bases such as triethylamine, diisopropylethylamine, pyridine, DABCO, DMAP and DBU; organic lithium compounds such as n-butyl lithium, sec-butyl lithium and t-butyl lithium; lithium amides such as LDA and lithium bistrimethylsilylamide; Grignard reagents such as ethyl magnesium bromide; alkali metal hydroxides such as sodium hydroxide and potassium hydroxide; alkali metal carbonates such as sodium carbonate and potassium carbonate; and metal hydrides such as sodium hydride and calcium hydride. These bases can be used alone or combining two or more.

An amount of a base used is in a range between 1 and 3 moles to a mole of pyrazolecarbonitrile. The reaction proceeds smoothly at a temperature from -10 Cto the boiling point of the solvent used.

Then, the obtained adduct [VII] is hydrolyzed in a solvent under an acidic condition to give the target 4-cyanoacetylpyrazole of Formula [I]. Examples of acids used for the hydrolysis include p-toluenesulfonic acid, p-toluenesulfonic acid hydrate, benzene sulfonic acid, sulfuric acid, hydrochloric acid and hydrogen bromide. An amount of an acid used is arbitrarily chosen in a range between 0.5 moles and excessive moles, preferably between 0.9 and 3 moles, to a mole of pyrazolecarboxylic acid halide.

Examples of solvents used for the hydrolysis reaction include water, alcohols such as methanol and ethanol; aqueous solvents such as water-methanol, water-ethanol, water-dioxane, water-acetone and water-THF; protic polar solvents such as acetone and THF; and aromatic hydrocarbons such as benzene or toluene. 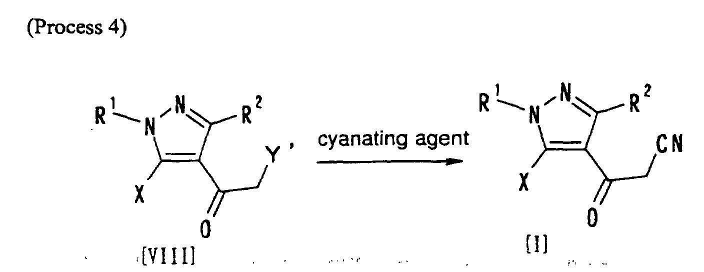 (In the equation, R¹, R², X and Y' are as defined above.)

A 4-cyanoacetylpyrazole [I] can be produced by reacting a haloacetylpyrazole [VIII] with a cyanating agent in a proper solvent.

Actual examples of cyanating agents include sodium cyanide, potassium cyanide, calcium cyanide, copper (I) cyanide and acetone cyanohydrin. An amount of the agent used can be arbitrarily chosen in a range between 0.5 mole and excessive moles, preferably between 1.0 and 10 moles, to a mole of haloacetylpyrazole.

Preferred solvents used for the cyanation reaction are polar solvents. Their actual examples include alcohols such as methanol, ethanol, n-propanol, isopropanol, n-hexanol, ethylene glycol, diethylene glycol and glycerin; and DMF, N,N dimethylacetamide, DMSO, THF, 1,2-dimethoxyethane, 1,4-dioxane and acetonitrile. They can be used with water, if required.

A reaction temperature is usually in a range between -30 Cand the boiling point of the solvent used, preferably -10 C and room temperature.

Next, a process for the preparation of a pyrazolecarboxylic acid, a starting material for the above Processes 1 and 2, is described.

According to the process of the present invention, it is possible to produce a target carboxylic acid in high yield when a reaction is carried out using water as a reaction solvent and only hydrogen peroxide as an oxidizing agent in the presence of a base such as sodium hydroxide or potassium hydroxide. A hydroxide used is at an amount required to keep the reaction system alkaline. It is about 2 to 5 equivalents to an amount of the aldehyde.

A reaction temperature is from 30 Cto the boiling point, preferably 40 C to 60 C.

A reaction time is 3 to 5 hours including a maturing time after the completion of hydrogen peroxide dropping. After the reaction is completed, the product is treated with concentrated hydrochloric acid (pH = 1). Filtration of the deposited crystals gives the target compound.

Upon the completion of the reaction, the reaction product is isolated and purified by ordinary means of synthetic organic chemistry to give a target compound. The structure of the target compound can be determined by measuring various spectra, such as those of ¹H-NMR, IR and MASS, and by other methods.

### Best Forms to Implement the Invention:

The present invention is described in detail in reference to Examples. The present invention is not however restricted to the examples.

### Example 1

Preparation of 5-chloro-4-cyanoacetyl-1-methyl-3-trifluoromethylpyrazole

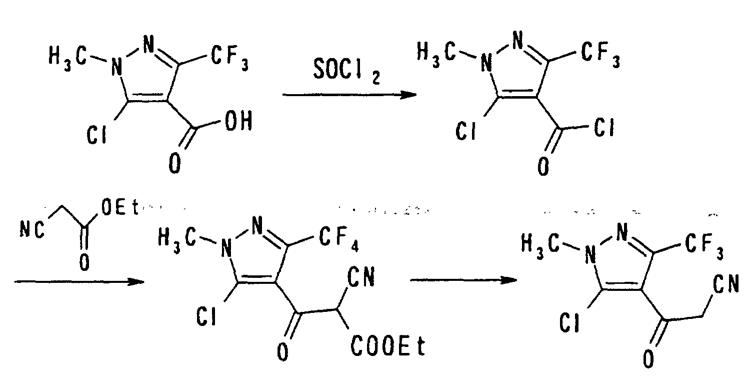

5g of 5-chloro-1-methyl-3-trilluooomethylpyrazol-4-carboxylic acid was reacted with 3.9g of thionyl chloride to give 5-hloro-1-methyl-3-trifluoromethylpyrazol-4-carboxylic acid chloride. The chloride obtained was dissolved in 40 ml of toluene, and 0.25g of 4-dimethylaminopyridine and 4.95g of ethyl cyanoacetate were added. The resulting solution was heated to 60 C and 4.4g of triethylamine was added with stirring. The solution was heated for another an hour after the completion of the addition.

The reaction solution was cooled to room temperature, and 50 ml of 7% hydrochloric acid solution was added on cooling. The toluene layer was separated. 15ml of 28% sodium hydroxide solution and 35 ml of water were added to the toluene solution. The resulting solution was heated at reflux temparature for 5 hours, and cooled. The aqueous layer was separated. Concentrated hydrochloric acid was added to the layer for decarboxylation. The resulting crystals separated by filtration, washed with 50 ml of water and dried to give 4.27g of the target compound, 5-chloro-4-cyanoacetyl-1-methyl-3-trifluoromethylpyrazole. Melting point: 105 - 107 C.¹H-NMR (CDCl₃, äppm): 3.80 (s, 3H), 4.30 (s, 2H).

### Example 2

Preparation of 5-chloro-4-cyanoacetyl-1-methyl-3-trifluoromethylpyrazole

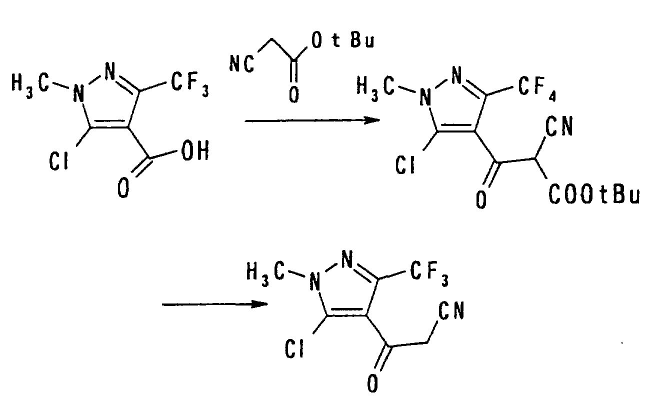

1.0g of 5-chloro-1-methyl-3-trifluoromethylpyrazol-4-carboxylic acid, 0.25g of t-butyl cyanoacetate, 0.71g of diethylcyanophosphonate and 1.2g of triethylamine were mixed in 10 ml of dimethylformamide, and stirred overnight at room temperature. Water was added to the reaction mixture to extract with ethyl acetate. The organic layer was washed with 10% of sulfuric acid solution and with saturated aqueous sodium carbonate, and dried over anhydrous magnesium sulfate. The solvent was distilled out under reduced pressure. 1.3g of the resulting oil was dissolved in 10ml of toluene, and 0.1g of p-toluenesulfonic acid hydrate was added. The solution was heated at reflux temparature for 3 hours. The reaction solution was washed with water. Toluene was distilled out under reduced pressure. The crude product obtained were washed with ether, and dissolved in 5 ml of chloroform. 2 ml of 5% sodium hydroxide solution added to the chloroform solution with stirring. After the solution was stirred sufficiently, the aqueous layer was separated and neutralized with hydrochloric acid. The resulting crystals separated, washed with water sufficiently and dried to give 0.15g of the target compound, 5-chloro-4-cyanoacetyl-1-methyl-3-trifluoromethylpyrazole. Melting point: 105 - 107.5 C.

### Example 3

Preparation of 5-chloro-4-cyanoacetyl-1-methyl-3-trifluoromethylpyrazole

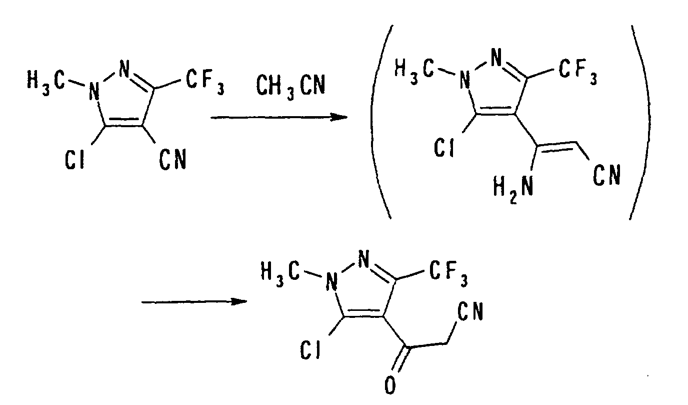

A mixed solution of 1g of 5-chloro-4-cyano-1-methyl-3-trifluoromethylpyrazole, 0.22g of acetonitrile, 0.035g of t-butyl alcohol and 15 ml of diethyl ether was dropped in a suspension of 0.21g of 60% sodium hydride in 10 ml of diethyl ether at 0 Cunder the nitrogen atmosphere, followed by a reaction for 18 hours at room temperature. The reaction solution was poured into ice-water, and extracted with ethyl acetate. The extract was washed with water and dried over anhydrous magnesium sulfate. Then, the solvent was distilled out under reduced pressure. The residue obtained was dissolved in 20 ml of chloroform, and 10 ml of 3N hydrochloric acid was added to it. The resulting two layers solution was stirred at room temperature for 18 hours. The chloroform layer was separated, washed with water and dried over anhydrous magnesium sulfate. The solvent was distilled out under reduced pressure. The residue obtained was purified through a silica gel column chromatography (n-hexane/ethyl acetate = 1/1) to give 0.5g of the target compound, 5-chloro-4-cyanoacetyl-l-methyl-3-trifluoromethylpyrazole. Melting point: 106 - 108 C.

### Example 4

Preparation of 5-chloro-4-cyanoacetyl-1-methyl-3-trifluoromethylpyrazole

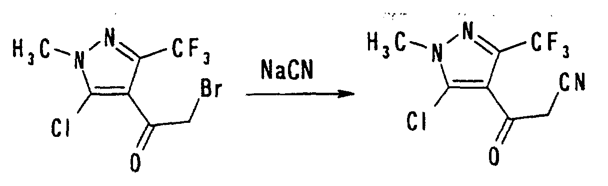

95g of 4-bromoacetyl-5-chloro-1-methyl-3-trifluoromethylpyrazole was dissolved in 600 ml of ethanol, and a solution of45.6g of sodium cyanide in 270 ml of water was dropped into it at 0 C. The reaction solution was warmed up to room temperature and stirred at the same temperature for 12 hours. The reaction mixture was poured into ice-water, and concentrated hydrochloric acid was carefully added to adjust pH to about 2. The aqueous layer was extracted with ethyl acetate. The organic layer was dried over anhydrous magnesium sulfate. The solvent was distilled out under reduced pressure. The oily product obtained was crystallized from a mixed solvent of ethyl acetate and hexane to give 66.8g of the target compound. 5-chloro-4-cyanoacetyl-1-methyl-3-trifluoromethylpyrazole. Melting point: 103 - 105 C

### Example 5

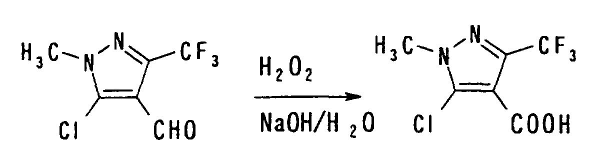

10g of 5-chloro-1-methyl-3-trifluoromethylpyrazol-4-carboaldehyde was added to an aqueous solution of 5g of sodium hydroxide (2 equivalents to the amount of the aldehyde) in 50 ml of water. While stirring the resulting solution vigorously, 34.4g of 35% hydrogen peroxide was added, in eight portions, over 1.5 hours at 40 Cto 50 CAfter the completion of the dropping, the solution was stirred for 30 minutes, cooled to 15 Cand adjusted pH to 1 with concentrated hydrochloric acid. The crystals obtained were sufficiently washed with water and dissolved in ether. The ether solution was dried over anhydrous magnesium sulfate and further treated with active carbon. Ether was distilled out under reduced pressure to give 8.6g of the target compound. Melting point: 197.5 - 199.5 C. Yield: 80.0%.

### Applicability in Industry:

The 4-cyanoacetylpyrazoles of the present invention, represented by Formula (1), are useful as intermediates for producing agricultural chemicals and drugs, for example for producing insecticides and acarcides described in Japanese Patent Laid-open No. Hei 11-269173 and Patent Application No. 2000-178334.

According to the present invention, 4-cyanoacetylpyrazoles useful as intermediates for producing compounds with insecticidal and acaricidal activities can be produced by an industrially advantageous process using easily available starting materials.

The present invention does not use at all reaction solvents, such as toluene or ethylene dichloride; heavy metal oxidizing agents or heavy metal catalysts, which known processes have adopted. Use of water as a reaction solvent and only hydrogen peroxide as an oxidizing agent allows an industrially advantageous process to produce substituted pyrazole-4-carboxylic acids.

## Claims

1. A process for the preparation of a compound represented by Formula [I] 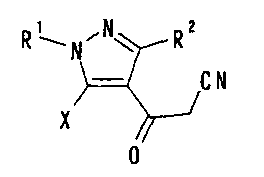 (wherein, R¹ is alkyl having 1 to 6 carbons, R² is alkyl having 1 to 6 carbons or haloalkyl having 1 to 6 carbons, and X is hydrogen, halogen or alkyl having 1 to 6 carbons), which comprises a process for producing a compound represented by Formula [TV] 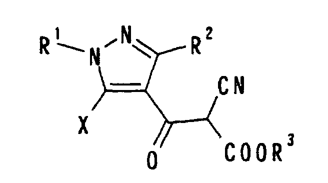 (wherein, R¹, R² and X are as defined above and R³ is alkyl having 1 to 6 carbons) by reacting a pyrazolecarboxylic acid halide represented by Formula [II] 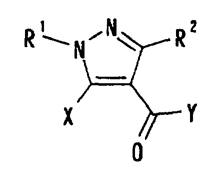 (wherein, R¹, R² and X are as defined above, and Y is halogen) with a cyanoacetate ester represented by Formula [III] 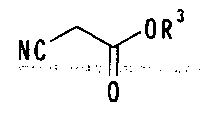 (wherein, R³ is as defined above) in the presence of a base, and a process for the hydrolysis and decarboxylation of the obtained compound of Formula [IV].

2. A process for the preparation of a compound of Formula [I] which comprises a process for producing a compound of [IV] by reacting a pyrazolecarboxylic acid of Formula [V] 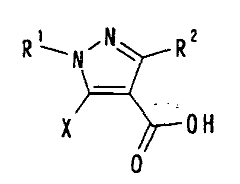 (wherein, R¹, R² and X are as defined above) with a cyanoacetate ester of Formula [III] in the presence of a condensing agent, and a process for the hydrolysis and decarboxylation of the obtained compound of Formula [IV].

3. A process for the preparation of a compound of Formula [I] which comprises a process for producing a compound of [VII] 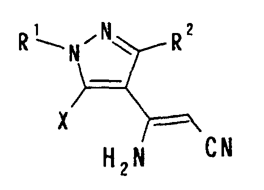 (wherein, R¹, R² and X are as defined above) by reacting a pyrazolecarbonitrile of Formula [VI] 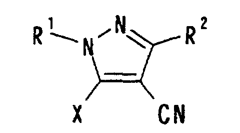 (wherein, R¹, R² and X are as defined above) with acetonitrile in the presence of a base, and a process for the hydrolysis of the obtained compound of Formula [VII].

4. A process for the preparation of a compound of Formula [I] which comprises reacting a haloacetylpyrazole of Formula [VIII] 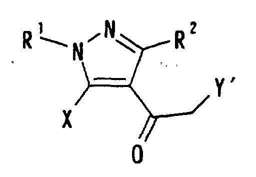 (wherein, R¹, R² and X are as defined above, and Y' is halogen) with a cyanating agent

5. A process for the preparation of a pyrazolecarboxylic acid of Formula [X] 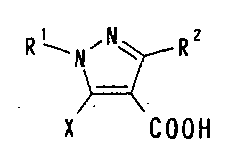 (wherein, R¹, R² and X are as defined above) which comprises reacting a compound represented by Formula [IX] 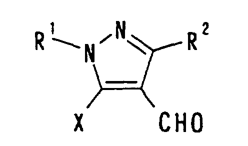 (wherein, R¹, R² and X are as defined above) with hydrogen peroxide in the presence of a base.

6. A process according to Claim 5 in which the base is sodium hydroxide or potassium hydroxide.

7. A process according to Claim 5 in which 2 equivalents or more of the base to an amount of Compound [IX] is added to keep the reaction system alkaline.
